# DEMANDE DE BREVET EUROPEEN

(11) **EP 0 604 248 A1**
(43) Date de publication de la demande: **29.06.1994**
(21) Numéro de dépôt: 93402816.8
(22) Date de dépôt: 19.11.1993
(51) Int. Cl.: A61K 31/365

(54) **Utilisation du 5-methoxypsoralene dans le traitement du sida**

(30) Priorité: 20.11.1992 FR 9213953
(71) Demandeur: Goupil, Jean-Jacques, F-92100 Boulogne (FR)
(72) Inventeur: Goupil, Jean-Jacques, F-92100 Boulogne (FR)
(74) Mandataire: Petit, Hélène

(57) **Abrégé**

La présente invention concerne l'utilisation du 5-méthoxypsoralène pour l'obtention de médicaments destinés au traitement d'états pathologiques dus à l'infection d'un individu par des virus du type HIV, et plus particulièrement du type HIV-1 et HIV-2.

L'invention vise également une méthode de traitement par voie extra-corporelle du sang de patients susceptibles d'être porteurs de virus du type HIV, comprenant une étape d'exposition aux rayons UVA du sang mis en circulation extra-corporelle, ce sang ayant été préalablement traité avec du 5-méthoxypsoralène.

## Description

La présente invention a pour objet l'utilisation du 5-méthoxypsoralène dans le cadre du traitement du SIDA, et plus particulièrement pour la mise en oeuvre de méthodes de traitement par voie extra-corporelle du sang de patients susceptibles d'être porteurs de virus du type HIV.

Les psoralènes sont des furocoumarines naturelles que l'on trouve dans les plantes. Ces furocoumarines sont connues comme étant des molécules capables d'inactiver photochimiquement les virus à ADN et à ARN.

Ces molécules sont notamment utilisées dans le cadre du traitement du psoriasis, en association à l'exposition de la peau des patients ainsi traités aux rayons ultraviolets du type A (UVA); on parle alors de puvathérapie (Parrish J.A. et al, 1974, N. ENGL. J. MED. 291: 1207-11, et autres).

Une de ces furocoumarines, le 8-méthoxypsoralène, a été utilisée dans le cadre de la mise en oeuvre d'une méthode appelée "photophorèse" et consistant au traitement par voie extra-corporelle des leucocytes du sang d'un patient par le 8-méthoxypsoralène en présence d'UVA (Edelson R.L., 1989, Progress in Dermatology, 23: 1-6). L'utilisation du seul 8-méthoxypsoralène par photophorèse a fait l'objet de la demande de brevet internationale WO 90/07952 du 10 janvier 1990, dans le cadre du traitement de patients présentant une infection virale et possédant un nombre anormalement faible de lymphocytes ou de leucocytes, notamment dans le cadre du traitement du SIDA.

La présente invention découle de la découverte faite par les inventeurs du fait que le 5-méthoxypsoralène provoque *in vitro,* en association avec les UVA, une réduction sensible du pouvoir infectieux des virus responsables du SIDA, à savoir des virus du type HIV.

Ainsi la présente invention a pour but de fournir de nouvelles compositions pharmaceutiques et de nouvelles méthodes de traitement du sang de patients par voie extra-corporelle dans le cadre du traitement du SIDA.

La présente invention a pour objet l'utilisation du 5-méthoxypsoralène pour l'obtention de médicaments destinés au traitement d'états pathologiques dus à l'infection d'un individu par des virus du type HIV, et plus particulièrement du type HIV-1 et HIV-2.

De tels médicaments sont caractérisés en ce qu'ils comprennent une quantité de 5-méthoxypsoralène, en association avec un véhicule pharmaceutiquement acceptable.

Le traitement des patients porteurs de virus du type HIV est avantageusement réalisé par la méthode dite photophorèse mentionnée ci-dessus; cette méthode de traitement du sang par voie extra-corporelle comprend une étape d'exposition aux rayons UVA d'une quantité déterminée de sang mis en circulation extra-corporelle, ce sang ayant reçu préalablement une quantité déterminée de 5-méthoxypsoralène. Le sang des patients ainsi traité est ensuite administré à ces derniers.

La présente invention a pour objet une méthode de traitement par voie extra-corporelle du sang de patients susceptibles d'être porteurs de virus du type HIV, caractérisée en ce qu'une quantité déterminée de 5-méthoxypsoralène est ajoutée au sang mis en circulation extra-corporelle, puis ce sang contenant du 5-méthoxypsoralène est traité aux rayons ultraviolets du type A.

Selon un mode de réalisation particulièrement avantageux de l'invention, le médicament contenant le 5-méthoxypsoralène est administré aux patients par voie orale ou parentérale, environ deux heures avant prélèvement d'une fraction du sang de ce patient et traitement de ce sang par les rayons UVA.

La quantité de 5-MOP administrée au patient peut varier entre 1,5 mg et 10 mg par kilogramme de poids corporel.

A ce titre l'invention concerne une méthode de traitement par voie extra-corporelle du sang de patients susceptibles d'être porteurs de virus du type HIV, caractérisée en ce qu'une quantité déterminée du sang de ces patients, auxquels a été préalablement administrée une quantité déterminée de 5-méthoxypsoralène, est mise en circulation extra-corporelle et traitée aux rayons ultraviolets du type A.

Le cas échéant, une quantité déterminée de 5-méthoxypsoralène est ajoutée à la fraction de sang mis en circulation extra-corporelle, préalablement à l'étape de traitement par les rayons UVA de la méthode précédente.

A titre illustratif, les quantités de sang mis en circulation extra-corporelle pour être traitées selon les méthodes décrites ci-dessus, sont d'environ 500 ml à environ deux litres.

La dose en UVA utilisée dans les méthodes susmentionnées, est d'environ 0,5 à environ 3, voire 4 Joules/cm² (une dose de 2 Joules/cm² correspondant à une irradiation de 1,5 mWatt/cm² pendant 17 mn). Ces doses peuvent être largement dépassées suivant la tolérance individuelle des patients pour obtenir le meilleur résultat.

Les méthodes de traitement par photophorèse telles que décrites ci-dessus, peuvent également être couplées au traitement par puvathérapie des patients avant ou après que ces derniers ont été traités par photophorèse.

L'invention a également pour objet toute composition sanguine telle qu'obtenue par mise en oeuvre d'une des méthodes de traitement du sang décrites ci-dessus.

De telles compositions sanguines sont plus particulièrement caractérisées en ce qu'elles sont constituées de sang prélevé chez un individu susceptible d'être porteur de virus du type HIV, et contenant du 5-méthoxypsoralène, ce sang ayant été soumis à un rayonnement du type UVA.

A ce titre, l'invention concerne également un vaccin dirigé contre les virus du type HIV, et préparé à partir des compositions sanguines décrites ci-dessus.

Ce vaccin peut être fabriqué selon la méthode suivante. On prélève du sang d'un malade atteint du SIDA, traité au 5-MOP ainsi que cela a été décrit précédemment, on concentre les lymphocytes et irradie le concentrat aux rayons UVA. On obtient des lymphocytes T4 morts et porteurs d'antigènes du virus HIV. On injecte ensuite cet échantillon sur un animal tel que le cheval, et on procède à la fabrication traditionnelle d'un vaccin qui sera constitué par les immunoglobulines extraites du sang du cheval.

L'invention sera davantage illustrée dans la description détaillée qui suit de l'effet du 5-méthoxypsoralène associé aux UVA, in vitro, sur le pouvoir infectieux de HIV-1.

### I - METHODES

Cette étude a été réalisée en 2 étapes :
- traitements du virus VIH-1 (ou HIV-1) par le 5-méthoxypsoralène (5-MOP) et les UVA.
- étude de l'infectivité résiduelle des échantillons traités à l'aide des tests sur cellules MT4 et sur lymphocytes activés.

### 1) TRAITEMENTS DU VIH-1:

Le virus a été traité par 10 µg/ml de 5-MOP, puis exposé aux UVA (1 à 2 Joules/cm²) de la façon suivante:
- 4 ml de virus VIH-1 ont été traités avec 40 µl de 5-MOP à 1 mg/ml puis exposés aux UVA (intensité de 1,5 mW/cm²) pendant 17 minutes (échantillon PUV 10),
- l'irradiation aux UVA a été faite avec la lampe PUVA 200 WALDMANN en déposant les flacons contenant le virus sur un lit de glace, afin d'éviter au maximum l'échauffement.

Les témoins pour cette expérience ont été les suivants :
- le virus VIH-1 utilisé, non traité (échantillon PUV 1),
- 4 ml du virus utilisé, traité uniquement par 40 µl de DMSO, afin de vérifier une éventuelle toxicité due au DMSO (échantillon PUV 2),
- le virus utilisé, traité avec 10 µg/ml de 5-MOP, mais sans irradiation UVA (échantillon PUV 6),
- le virus utilisé, traité uniquement par irradiation UVA pendant 17 minutes à 1,5 mW/cm² (échantillon PUV 5),
- le virus utilisé, sans traitement, gardé à la température de l'expérience, pendant toute la durée du traitement, afin de vérifier la perte d'infectivité due aux conditions de la manipulation (échantillon PUV 9).

### 2) RECHERCHE DU POUVOIR INFECTIEUX RESIDUEL DES ECHANTILLONS

a - Sur cellules MT4 :
   Des dilutions successives de chaque échantillon ont été testées en duplicata. 100 µl de chaque dilution ont été mis en contact 1 heure à 37°C avec 3.10⁵ cellules MT4 par puits de plaque COSTAR 24 puits. Les cellules ont été centrifugées afin d'éliminer le produit éventuellement toxique, puis resuspendues dans 1 ml du milieu de culture. Aux jours 3 et 6, 750 µl de surnageant ont été ôtés, remplacés par du milieu de culture frais et la concentration cellulaire ajustée à 3.10⁵ cellules par millilitre.
   Au jour 10, l'effet cytopathogène et la mortalité cellulaire ont été évalués par observation au microscope après coloration au bleu Trypan.
   Des dosages de l'activité transcriptase inverse ont été réalisés à partir des surnageants de culture conservés à chaque passage (jours 3, 6 et 10).
b - Sur lymphocytes humains activés :
   Un culot de 3.10⁶ lymphocytes préalablement stimulés à la PHA a été incubé avec 1 ml de chaque échantillon (de 10⁻¹ à 10⁻⁵ pour chaque échantillon, sauf pour l'échantillon PUV10 testé également non dilué) pendant 1 heure à 37°C. Puis les cellules ont été centrifugées et resuspendues à 10⁶ cellules par millilitre dans du milieu complet. Tous les 3 ou 4 jours, les cellules ont été comptées, réensemencées à 10⁶ cellules/ml et les surnageants conservés à -80°C. Un dosage de l'activité transcriptase inverse a été réalisé à partir des surnageants de culture à chaque passage.
c - Mesure de l'activité transcriptase inverse :
   L'activité transcriptase inverse a été dosée à partir d'1 ml de surnageant de culture concentré 100 fois par ultracentrifugation 5 mn à 95.000 rpm. Le culot a été resuspendu dans 10 µl de NTE contenant 0,1% de Triton X100. L'activité enzymatique a été révélée par l'addition de 40 µl du mélange réactionnel suivant: Tris 50 mM pH 7.9 ; KCl 20 mM; MgCl₂ 5 mM ; dithiotreitol 1 mM ; poly rA 0,05 D.O./ml ; oligo dT₁₂₋₁₈ 0,05 D.O./ml et ³HTTP 5 µCi.

Après 1 heure à 37°C, les produits acido-solubles ont été précipités par de l'acide trichloracétique 20%, filtrés sur Millipore 0,45 µm et la radioactivité B a été mesurée à l'aide d'un compteur à scintillation.

### II - RESULTATS

1) Infectivité résiduelle du VIH-1 traité 5-MOP et UVA: test sur cellules MT4:
   - l'effet du traitement par 10 µg/ml de 5-MOP associé à une irradiation UVA a été comparé aux données obtenues lors de traitements séparés 5-MOP (10 µg/ml) ou UVA,
   - les données obtenues pour le traitement UVA seul (échantillon PUV5) révèlent la présence d'une expression virale dans les cultures infectées par le VIH-1 traité UVA (dilution 10^{-5.5}), expression détectée par dosage d'antigène p25 dans les surnageants de cultures et par observation d'un effet cytopathogène sur les cellules du test. L'activité transcriptase inverse des surnageants est négative pour une infection à la dilution 10^{-5.5} mais positive à la dilution 10⁻⁵. Etant donné la sensibilité inférieure du test transcriptase inverse comparativement au dosage p25, on peut considérer que le titre de l'échantillon PUV5 est de 10^{5.5} UI/ml. Le traitement UVA n'a donc aucune action sur le virus aux doses d'UVA utilisées, comparativement au témoin PUV9,
   - l'analyse des résultats observés pour le traitement 5-MOP seul à 10 µg/ml montre une perte d'infectivité de 0,5 log puisque le titre du virus traité (échantillon PUV6) sur cellules MT4 est de 10⁵ UI/ml au lieu de 10^{5.5} UI/ml pour le témoin PUV9,
   - enfin, lorsque l'on analyse les données obtenues pour le virus ayant subi la combinaison du traitement (5-MOP à 10 µg/ml et UVA = échantillon PUV10), on constate que cette association conduit à une réduction du titre infectieux de 2,5 log comparativement au témoin PUV9. Cette réduction est donc supérieure de 2 log à celle observée pour le traitement 5-MOP à 10 µg/ml seul (facteur de réduction = 0,5).
2) Infectivité résiduelle du VIH-1 traité au 5-MOP et aux UVA: test sur cultures primaires de lymphocytes humains:
   Cette fois le pouvoir infectieux a été mesuré sur des lymphocytes humains (PBL) activés, cellules cibles naturelles du virus.

La recherche d'une expression virale par ces cellules a été suivie par mesure d'une activité transcriptase inverse dans les surnageants de cultures tous les 3 ou 4 jours pendant 28 jours.
- l'irradiation aux UVA à elle seule (échantillon PUV5) ne possède aucun pouvoir inactivant sur le virus. Le titre du virus traité UVA est, en effet, lui aussi identique à celui du témoin PUV9 (titre = 10² UI/ml),
- l'analyse des résultats de l'échantillon PUV10 retesté pur dans ces conditions permet de démontrer une inactivation virale de 3 log induite par la combinaison de traitement 5-MOP à 10 µg/ml et UVA (titre PUV10 = 100 UI/ml).

### III- Conclusion

Seul le traitement 5-MOP à 10 µg/ml et UVA provoque une réduction du pouvoir infectieux du virus d'environ 3 log, les traitements témoins 5-MOP seul ou UVA seul étant sans effet.

## Revendications

1. Utilisation du 5-méthoxypsoralène pour l'obtention d'un médicament destiné au traitement d'états pathologiques dus à l'infection d'un individu par des virus du type HIV, et plus particulièrement du type HIV-1 et HIV-2.

2. Utilisation du 5-méthoxypsoralène selon la revendication 1, pour l'obtention d'un médicament destiné au traitement par voie extra-corporelle du sang de patients susceptibles d'être porteurs de virus du type HIV, ce traitement comprenant une étape d'exposition aux rayons UVA d'une quantité déterminée de sang mis en circulation extra-corporelle, ce sang contenant une quantité déterminée de 5-méthoxypsoralène.

3. Utilisation du 5-méthoxypsoralène selon la revendication 2, caractérisée en ce qu'une quantité déterminée de 5-méthoxypsoralène est ajoutée au sang mis en circulation extra-corporelle, puis ce sang contenant du 5-méthoxypsoralène est traité aux rayons ultraviolets du type A.

4. Utilisation du 5-méthoxypsoralène selon la revendication 2 ou la revendication 3, caractérisée en ce qu'une quantité déterminée du sang de ces patients, auxquels a été préalablement administrée une quantité appropriée de 5-méthoxypsoralène, notamment de l'ordre de 1,5 mg à 10 mg par kg de poids corporel du patient, est mise en circulation extra-corporelle et traitée aux rayons ultraviolets du type A.

5. Composition sanguine telle qu'obtenue par exposition aux rayons UVA d'une quantité déterminée de sang prélevé chez des patients susceptibles d'être porteurs de virus du type HIV, ce sang contenant une quantité déterminée de 5-méthoxypsoralène.

6. Vaccin dirigé contre les virus du type HIV caractérisé en ce qu'il est préparé à partir d'une composition selon la revendication 5.
